# EUROPEAN PATENT SPECIFICATION

(11) **EP 0 804 131 B1**
(45) Date of publication and mention of the grant of the patent: **28.12.2005**
(21) Application number: 96903391.9
(22) Date of filing: 04.01.1996
(51) Int. Cl.: A61F 9/00

(54) **APPARATUS FOR FOLDING A DEFORMABLE INTRAOCULAR LENS**
VORRICHTUNG ZUM FALTEN EINER DEFORMIERBAREN INTRAOKULAREN LINSE
APPAREIL POUR PLIER UN CRISTALLIN ARTIFICIEL DEFORMABLE

(30) Priority: 04.01.1995 US 368792
(43) Date of publication of application: 05.11.1997
(73) Proprietor: Staar Surgical Company, Monrovia, CA 91016 (US)
(72) Inventor: Feingold, Vladimir, Laguna Niguel, CA 92677 (US)
(74) Representative: Cawdell, Karen Teresa
(86) International application number: PCT/US1996/000264
(87) International publication number: WO 1996/020662

(56) References cited:
- WO-A-94/07436
- US-A- 5 275 604

## Description

### FIELD OF THE INVENTION

This invention relates to an apparatus for the injection of a deformable intraocular lens into the eye. Specifically, the present invention relates to injecting devices, and cartridges for inserting deformable intraocular lens into the eye.

### BACKGROUND OF THE INVENTION

Intraocular lenses have gained wide acceptance in replacement of human crystalline lenses after a variety of cataract removal procedures. The human crystalline lens is generally recognized to be a transparent structure having a thickness of about five (5) millimeters and a diameter of about nine (9) millimeters. The lens is suspended behind the iris by zonula fibers which connect the lens to the ciliary body. A lens capsule surrounds the lens, the front portion of the capsule being commonly known as the anterior capsule and the back portion commonly known as the posterior capsule.

Numerous procedures for the removal of cataracts have been developed in which the lens is removed from the eye and replaced by an artificial lens implant. The extraction procedure may generally be categorized as intracapsular (in which the lens is removed together with the lens capsule) and extracapsular (in which the anterior capsule is removed with the lens, and the posterior capsule is left intact).

Since Ridley implanted the first artificial lens in about 1949, the problems associated with cataract extraction and lens implantation have received a great deal of attention from ophthalmic surgeons. Various types of artificial lenses have been proposed, and appropriate surgical procedures have been developed which strive to reduce patient discomfort and to reduce postoperative complications. Reference is made in this connection to Pseudophakos by N. Jaffe et al.; "History of Intraocular Implants" by D.P. Choyce (Annals of Ophthalmology, October 1973); U.S. Patent No. 4,251,887 issued to Anis on February 24, 1981; U.S. Patent No. 4,092,743 issued to Kelman on November 8, 1977; "Comparison of Flexible Posterior Chamber Implants", presented at the American Intraocular Implant Society Symposium April 23, 1982, by Charles Berkert, M.D.; and "the Simcoe Posterior Lens" (Cilco, Inc. 1980); U.S. Patent No. 4,573,998 issued to Mazzocco on March 4, 1986, U.S. Patent No. 4,702,244 issued to Mazzocco on October 27, 1987; and U.S. Patent No. 4,715,373 issued to Mazzocco et al. on December 29, 1987.

Of particular interest in the context of the present invention is the development of surgical techniques requiring relatively small incisions in the ocular tissue for the removal of cataracts as disclosed in U.S. Patent No. 4,002,169 and U.S. Patent No. 3,996,935. A number of skilled artisans have disclosed intraocular lens structures comprising an optical zone portion generally made of rigid materials such as glass or plastics suitable for optical use.

However, one of the principal disadvantages of the conventional rigid intraocular lens is that implantation of the lens requires large incisions in the ocular tissue. This type of surgical procedure leads to a relatively high complication rate, among other disadvantages. For instance, the serious dangers associated with implantation of a rigid lens structure include increased risk of infection, retinal detachment, and laceration of the ocular tissue, particularly with respect to the pupil.

Accordingly, those skilled in the art have recognised a significant need for surgical tools for implantation of deformable intraocular lens structure which afford the clinical advantages of using relatively small incision techniques, which provide a safer and more convenient surgical procedure. In particular, those skilled in the art of deformable intraocular lenses and method and devices for implantation, have also recognised a significant need for surgical tools which do not require widening of the wound made in the ocular tissue during or after implantation, but will deform the intraocular lens to a predetermined cross section in a stressed state and which allow the ophthalmic surgeon to inspect the lens prior to implantation without manipulation in the eye. The present invention fulfills these needs.

The present invention was derived by improving the methods and devices in the above-identified patents, specifically the methods of US Patent No. 4573998 and the devices of US Patent No. 4702244.

US Patent 5275604 to Rheinish et al is particularly relevant with respect to the invention disclosed herein. US Patent No. 5275604 to Rheinish et al. discloses an apparatus and method for deforming and inserting a flexible intraocular lens into an eye suitable for use with lenses having radial flange or projecting filament haptics. The apparatus includes a duct with a pair of internal grooves configured to engage peripheral edges of a lens, curling the lens as it is advanced along the longitudinal duct axis from an inlet to an outlet of the duct.

WO 95 07 436 to Feingold is also particularly relevant with respect to the present invention. WO 95 07436 discloses a contoured duct apparatus for curling and inserting a flexible intraocular lens into an eye, comprising a holder having a receiver, a plunger, and a lens holder of the hinged type lens microcartridge.

### SUMMARY OF THE INVENTION

An object of the present invention is to provide an improved intraocular lens microcartridge.

Another object of the present invention is to provide a intraocular lens microcartridge for use with a surgical device for implantation of a deformable intraocular lens into the eye through a relatively small incision made in the ocular tissue, said lens microcartridge comprising a lens holder portion having a lens receiving portion for receiving and holding the deformable intraocular lens, and a nozzle portion connected to and extending from said lens holder portion, said lens holder portion and said nozzle portion having a continuous passageway extending therethrough.

A further object of the present invention is to provide a intraocular lens microcartridge for use with a surgical device for implantation of a deformable intraocular lens into the eye through a relatively small incision made in the ocular tissue, the lens microcartridge comprising a lens holder portion having a lens receiving portion for receiving and holding the deformable intraocular lens, and a nozzle portion connected to and extending from said lens holder portion and having a tapering configuration, the lens holder portion and the nozzle portion having a continuous passageway extending therethrough.

Another object of the present invention is to provide a cartridge having a pair of opposed converging grooves for folding the intraocular lens.

A further object of the present invention is to provide a cartridge having a configuration to accept the intraocular lens in a flat configuration with a pair of opposed converging grooves for folding the intraocular lens.

An even further object of the present invention is to provide a cartridge having a funnel-type configuration for further folding the intraocular lens.

Another even further object of the present invention is to provide a cartridge having a funnel-type configuration with a pair of opposed converging grooves for further folding the intraocular lens.

The present invention is directed to devices for implantation of intraocular lenses into the eye. In particular, the present invention is directed to hingeless type intraocular lens microcartridges.

A surgical device according to the present invention includes the combination of a lens holder and a holder for the lens holder or lens microcartridge. The preferred lens microcartridge comprises the combination of a lens receiver and an implantation nozzle.

The lens holder is a hingeless type microcartridge into which a lens is carefully loaded prior to being placed in the receiver of the surgical implantation device.

The lens holder is inserted into a holder (i.e. surgical implantation device) having means for driving or manipulating the lens from the lens holder into the eye. In the preferred embodiment, the holder is provided with a plunger for driving the lens from the lens holder into the eye. Further, the holder is configured to receive the microcartridge having a nozzle of the hinged or hingeless version.

The preferred holder can include means to prevent the microcartridge from rotating within the holder, and means for preventing the plunger from rotating within the holder. The means for preventing the plunger from rotating within the holder can be defined by providing the plunger and a sleeve within the holder with a particular cross-sectional shape that prevents rotation, for example, a half-circle shape.

The preferred holder includes a plunger with a threaded cap cooperating with a threaded sleeve of the holder body for dialing the plunger forward within the holder for precise and accurate movement of the lens during the implantation process. The holder is configured so that the plunger can be moved a predetermined distance by sliding motion within the holder body followed by engagement of the threaded cap of the plunger with the threaded sleeve of the holder body to continue the forward progress of the plunger tip.

The preferred plunger tip is defined by a faceted tip having various surfaces for moving and manipulating the lens from the lens holder and within the eye. The tip is designed to provide a clearance between the tip and the inner surface of the passageway through lens holder to accommodate the trailing haptic and prevent damage thereto. Once the lens is inserted into the eye, the tip can be used to push and rotated the lens into proper position within the eye.

Operation of the present invention includes lubricating the surface of a deformable intraocular lens with a surgically compatible lubricant, and loading the lens into a microcartridge. The microcartridge is inserted into the holder with the plunger retracted.

The plunger is moved forward in a sliding manner by pushing the plunger forward while holding the holder body still. This action forces the lens from the tubular member portion of the microcartridge into the nozzle portion. At this point the threads of the threaded end cap of the plunger engage with the threads of the threaded sleeve. The threaded end cap is rotate slightly to engage the threads. The device is now ready for the implantation process.

The nozzle of the microcartridge is placed through a small incision in the eye. The threaded end cap of the plunger is rotated or dialed to further advance the lens forward through the nozzle and into the eye. The threaded end cap is further dialed to exposed the tip of the plunger within the eye and push the lens into position. The tip can be used to also rotate the lens within the eye for positioning of the haptics.

A preferred embodiment of the cartridge according to the present invention is specifically configured to fold an intraocular lens that is initially loaded flat into the cartridge. This cartridge allows an intraocular lens to be potentially stored flat in the cartridge during shipment from the manufacturing facility to distributors and end users. Alternatively, an end user can simply load a packaged intraocular lens into the cartridge just prior to use or in preparation for the surgical procedure.

The cartridge includes a passageway defined by a set of opposed grooves that converge together folding the edges of the lens until the lens is sufficiently folded to pass through the nozzle portion of the cartridge. The tapering or converging nature of the passageway is preferably continuous so as to prevent any damage to the lens as it moves through the cartridge. In some embodiments, the grooves in the passageway converges along a portion of the length of the passageway, and in other embodiments the grooves converge along the entire length of the passageway through the lens holding portion and the nozzle portion of the cartridge. For example, in one embodiment, the grooves initially remain of constant size and cross-sectional shape until a transition portion of the passageway through the lens holding portion where the grooves converge folding the edges of the lens to a sufficient degree to allow the lens to pass through the passageway in the nozzle portion.

A further embodiment of the cartridge according to the present invention has a funnel-like construction wherein the passageway through the cartridge is funnel shaped to further fold the lens as it is moved through the cartridge. The cartridge can be provided with a set of opposed grooves located above a horizontal center plane of the cartridge that further fold the edges towards each other until the lens is fully folded to an extent to allow the lens to pass through the passageway of the nozzle portion. A preferred embodiment of the funnel-type cartridge is defined by an essentially oval-cross-sectional passageway that converges in a direction towards the nozzle portion. The oval shaped passageway is oriented so that the major axis is substantially aligned with the center plane of the lens.

### BRIEF DESCRIPTION OF THE DRAWINGS

Figure 1 is a perspective view of a prior art device with a lens holding microcartridge positioned in the device for implantation of deformable lens structures for placement in the eye;
Figure 2 is a perspective view of the surgical device depicted in Figure 1 with the plunger retracted, and with the lens holding microcartridge removed;
Figure 3 is a side view of the device depicted in Figure 2, with the plunger in the extended position;
Figure 4 is a side elevational view of the device shown in Figure 1;
Figure 5 is a detailed longitudinal cross-sectional view of the device shown in Figure 4;
Figure 6 is a detailed transverse cross-sectional view of the device, as indicated in Figure 5;
Figure 7 is a detailed end view of the device, as indicated in Figure 5;
Figure 8 is an enlarged detailed left side elevational view of the tip of the plunger in the spacial orientation as shown in Figure 1;
Figure 9 is an enlarged detailed end view of the tip shown in Figure 8;
Figure 10 is an enlarged detailed top planar view of the tip of the plunger;
Figure 11 is an enlarged detailed right side elevational view of the tip of the plunger in the spacial orientation, as shown in Figure 4;
Figure 12 is an enlarged detailed bottom view of the tip of the plunger in the spacial orientation, as shown in Figure 1;
Figure 13 is a perspective view of a lens for use in the present invention;
Figure 14 is a perspective view of another type of lens for use in the present invention;
Figure 15 is a side view of the lens shown in Figure 13;
Figure 16 is a perspective view of the prior art lens holding microcartridge in the open position to allow a lens to be loaded therein;
Figure 16A is another perspective view of the lens holding microcartridge in the open position;
Figure 17 is a rear end elevational view of the lens holding microcartridge in the open position;
Figure 18 is a front end elevational view of the lens holding microcartridge in the open position;
Figure 19 is a rear end elevational view of the lens holding microcartridge in the closed position;
Figure 20 is a front end elevational view of the lens holding microcartridge in the closed position;
Figure 20A is a detailed end view of the nozzle showing three (3) slots of different length equally spaced about the circumference of the tip;
Figure 20B is a detailed perspective view of the tip showing the three (3) slots of different length;
Figure 20C is a detailed side view showing the beveled tip;
Figure 21 is a top planar view of the lens holding microcartridge in the open position;
Figure 22 is a side elevational view of the lens holding microcartridge in the closed position;
Figure 23 is a rear end elevational view of the lens holding microcartridge in the closed position;
Figure 24 is a broken away side view of the device showing the lens holding microcartridge in relationship to the plunger in the retracted position;
Figure 25 is a broken away side view of the device showing the lens holding microcartridge in relationship to the plunger in a partially extended position;
Figure 26 is a broken away side view of the device showing the lens holding microcartridge in relationship to the plunger in a fully extended position;
Figure 27 is a perspective view showing the device positioning a deformable intraocular lens within the eye;
Figure 28 is a cross-sectional view of an eye showing the positioning of the deformable intraocular lens into position in the eye by the surgical device;
Figure 29 is a cross-sectional view of an eye showing the positioning of the deformable intraocular lens into a different position in the eye by the surgical device.
Figure 30 is a side elevational view of another prior art lens holding microcartridge provided with a beveled tip;
Figure 31 is a rear end elevational view of yet another prior art lens holding microcartridge provided with grooves in the passageway to facilitate folding the cartridge in an open position;
Figure 32 is a rear end elevational view of the prior art lens holding microcartridge provided with grooves in the passageway to facilitate folding the cartridge in a closed position;
Figure 33A is a front end elevational view of the nozzle of yet another prior art lens holding microcartridge; and
Figure 33B is a front end elevational view of the nozzle of yet another prior art lens holding microcartridge.
Figure 34 is a longitudinal cross-sectional view of a hingeless prior art intraocular lens microcartridge according to the present invention.
Figure 35 is a top view of the hingeless intraocular lens microcartridge, as shown in Figure 34.
Figure 36 is a partial broken away top view of the hingeless intraocular lens microcartridge, as shown in Figure 35.
Figure 37 is a cross-sectional view of the hingeless intraocular lens microcartridge at location 37-37, as shown in Figure 34.
Figure 38 is a cross-sectional view of the hingeless intraocular lens microcartridge as indicated at 38-38, as shown in Figure 34.
Figure 39 is a front elevational view of the hingeless intraocular lens microcartridge shown in Figures 34-37.
Figure 40 is a longitudinal cross-sectional view of the hingeless intraocular lens microcartridge, as shown in Figure 34, with an intraocular lens placed on top of the microcartridge ready for insertion therein.
Figure 41 is a rear elevational view of the microcartridge, as shown in Figure 40, with the intraocular lens not yet deformed for inserting into the microcartridge.
Figure 42 is a rear elevational view of the microcartridge, as shown in Figure 40, with the intraocular lens partial deformed and bent at the center being inserted into the microcartridge.
Figure 43 is a rear elevational view of the microcartridge, as shown in Figure 40, with the intraocular lens fully deformed and inserted inside the microcartridge.
Figure 44 is a partial view of an alternative tip portion of the microcartridge having a beveled end.
Figure 45 is a partial view of another alternative tip portion of the microcartridge having a heat deformed tip.
Figure 46 is a perspective view of an embodiment of the cartridge according to the present invention allowing for a deformable intraocular lens to be loaded in a flat orientation.
Figure 47 is a top planer view of the cartridge shown in Figure 46.
Figure 48 is a rear end elevational view of the cartridge shown in Figure 46.
Figure 49 is a transverse cross-sectional view of the cartridge as indicated in Figure 47.
Figure 50 is a transverse cross-sectional view of the cartridge as indicated in Figure 47.
Figure 51 is a transverse cross-sectional view of the cartridge as indicated in Figure 47.
Figure 52 is another preferred embodiment of the cartridge according to the present invention having a funnel-type configuration.
Figure 53 is a transverse cross-sectional view of the cartridge as indicated in Figure 52.
Figure 54 is a transverse cross-sectional view of the cartridge as indicated in Figure 52.
Figure 55 is a transverse cross-sectional view of the cartridge as indicated in Figure 52.
Figure 56 is a transverse cross-sectional view of the cartridge as indicated in Figure 52.
Figure 57 is a transverse cross-sectional view of the cartridge as indicated in Figure 52.

### DETAILED DESCRIPTION OF PREFERRED EMBODIMENTS

The present invention is directed to a device for implantation of deformable intraocular lens structures for surgical placement in the eye.

An device according to the prior art comprises a holder having a receiver, a lens holder that can be removably inserted into the receiver of the holder, and means such as a moveable plunger disposed within the holder to force and manipulate the lens from the lens holder into the eye.

The lens holder is defined by a lens holding microcartridge for receiving the lens structure. A microcartridge according to the prior art is a structure configured to be opened and closed. The microcartridge receives a lens having prescribed memory characteristics when in the open position, and performs the function of folding or deforming the lens structure into a condensed configuration when being closed. The microcartridge can be a structure having a passageway defined by a continuous walled annulus, and a lens could be inserted into the passageway from the end of microcartridge by compressing, rolling, folding, or combination of these techniques prior to insertion into the microcartridge.

Once a lens is positioned into the microcartridge, the microcartridge is positioned into a plunger device. The assembled device maintains the lens in its condensed configuration during insertion into the eye yet permits the deformed lens to return to its original configuration, size and fixed focal length once implanted in the eye, thereby providing a safe, convenient, and comfortable surgical procedure.

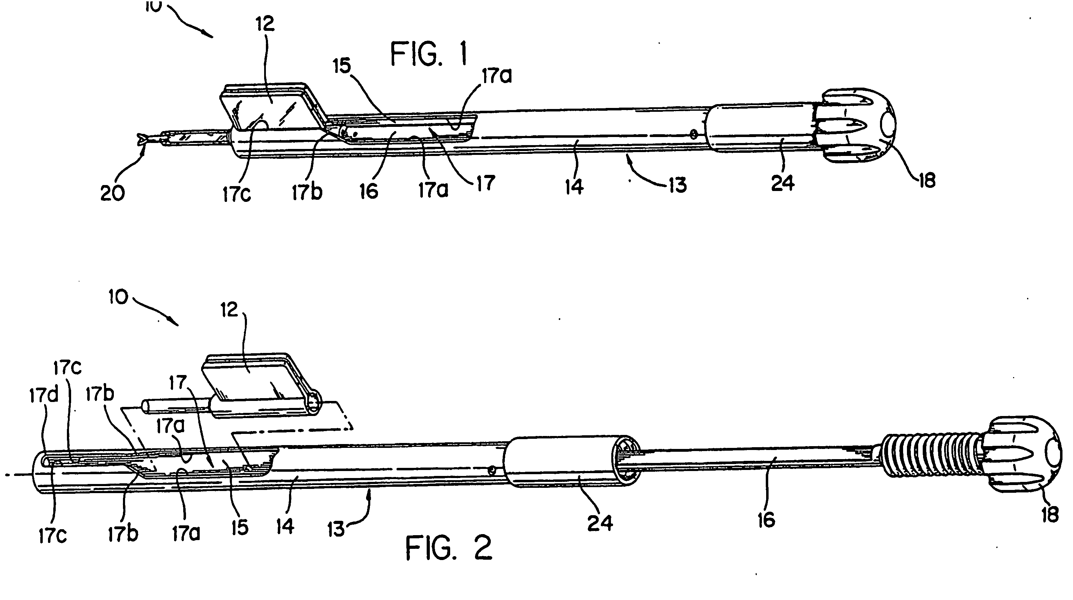

A deformable intraocular lens implantation device 10 according to the prior art is shown in Figures 1, 2 and 3. The implantation device comprises a microcartridge 12 disposed within a holder 13 comprising a holder body 14 with a receiver 15, and a moveable plunger 16. In Figure 1, the receiver 15 is defined by an opening 17 through the wall of the holder body 14 of the size and shape shown in Figures 1 and 2. The opening 17 is defined by parallel edges 17a, 17a, which are sufficiently spaced apart to allow the microcartridge 12 to be loaded into the receiver 15 of the holder 13, tapered edges 17b, clamping edges 17c, and stop edge 17d. In Figure 1, the microcartridge 12 is positioned in the receiver 15 between the clamping edges 17c with the plunger extending through the microcartridge 12 in a position, for example, after a lens implantation procedure.

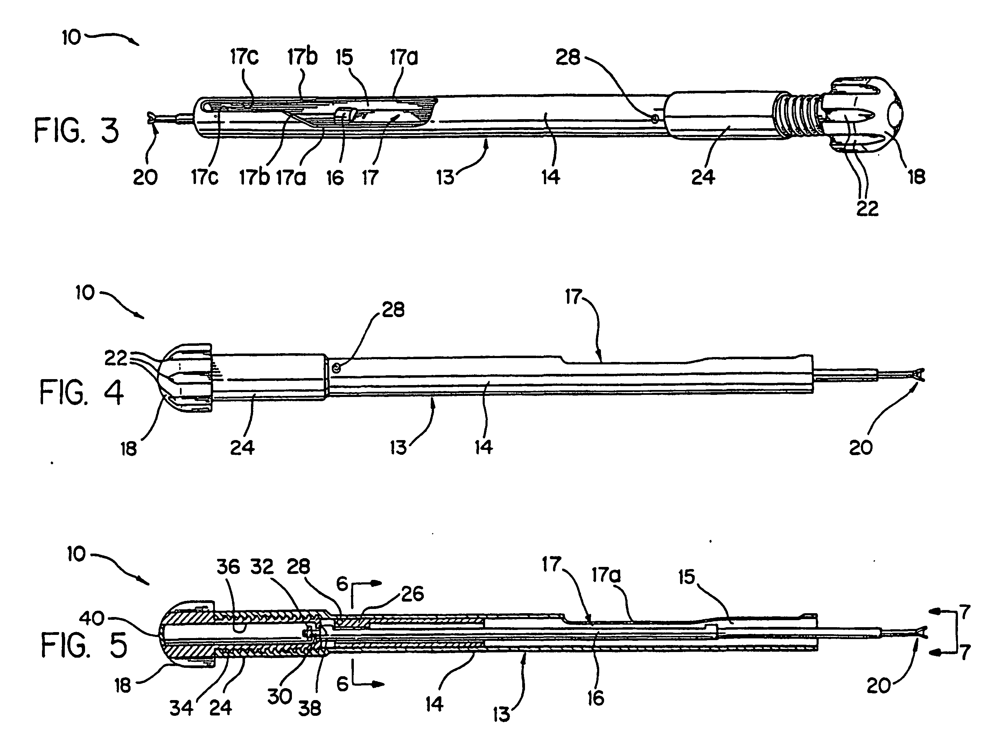

In Figure 2, the lens holding microcartridge 12 is shown removed from the holder 13 with the plunger 16 in a retracted position for allowing the microcartridge 12 containing a loaded lens and its haptic to be inserted within the holder 13. In Figure 3, the holder 13 is shown with the plunger 16 in the extended position without the microcartridge 12 for purposes of illustration of the components.

The plunger 16 is fitted with a threaded end cap 18 at one end, and fitted with a tip 20 at an opposite end. The threaded end cap 18 is provided with a plurality of grooves 22 to a allow a person to tightly grip the cap 18 with his or her finger tips. The threaded end cap 18 is received within a threaded sleeve 24 of the insert holder 14. The threaded end cap 18 can be a separate component attached to the insert holder 13, or integral therewith, as shown in the construction is Figure 5.

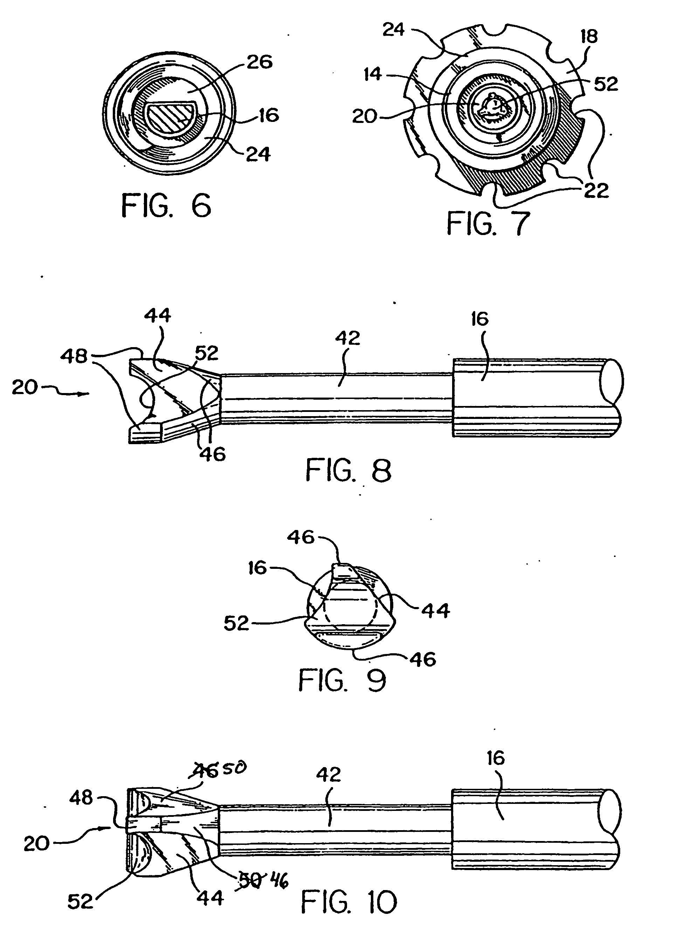

The plunger 16 is installed within the holder 13 in a manner to allow the plunger to be reciprocated therein. In the illustrated embodiment, the plunger 16 is supported for sliding movement within the holder 13 by guide 26, as shown in Figures 5 and 6. The outer dimension of the guide 26 is approximately the same size as the inner dimensions of the holder 13 to allow the guide to be inserted within the insert holder. During construction, the guide 26 is inserted within the holder 13, and locked into position by pin 28 inserted into a predrilled hole in both the wall of the holder 13 and guide 26.

The cross-sectional shape of the plunger 16 as well as the shape of the inner surface of the guide 26 are approximately a half-circle, as shown in Figure 6. This arrangement prevents the plunger 16 from rotating within the holder 13 to maintain the orientation of the tip 20 relative to the holder 13 during operation.

The threaded end cap 18 is connected to the plunger 16 in a manner to allow the threaded end cap 18 to be rotated relative to the plunger 16. For example, the left end of the plunger 16 (Figure 5) is provided with a threaded extension 30, which is secured to the threaded end cap 18 by a nut 32. Specifically, the threaded end cap 18 is manufactured with external threads 34 and a longitudinal center bore 36 that ends on the right side of the threaded end cap 18 leaving a wall 38.

The wall 38 is provided with a hole slightly larger than the outer diameter of the threaded extension 34 to allow the threaded end cap 18 to freely rotate on the plunger 16 while being secured to the end of the plunger 16. During construction, the nut 32 is inserted through the center bore 36 and threaded onto the extension 30 to secure the threaded end cap 18 to the plunger 16. A curved cap 40 is press fitted into the end of the center bore 36 to seal the center bore 36 to prevent debris from entering therein during use.

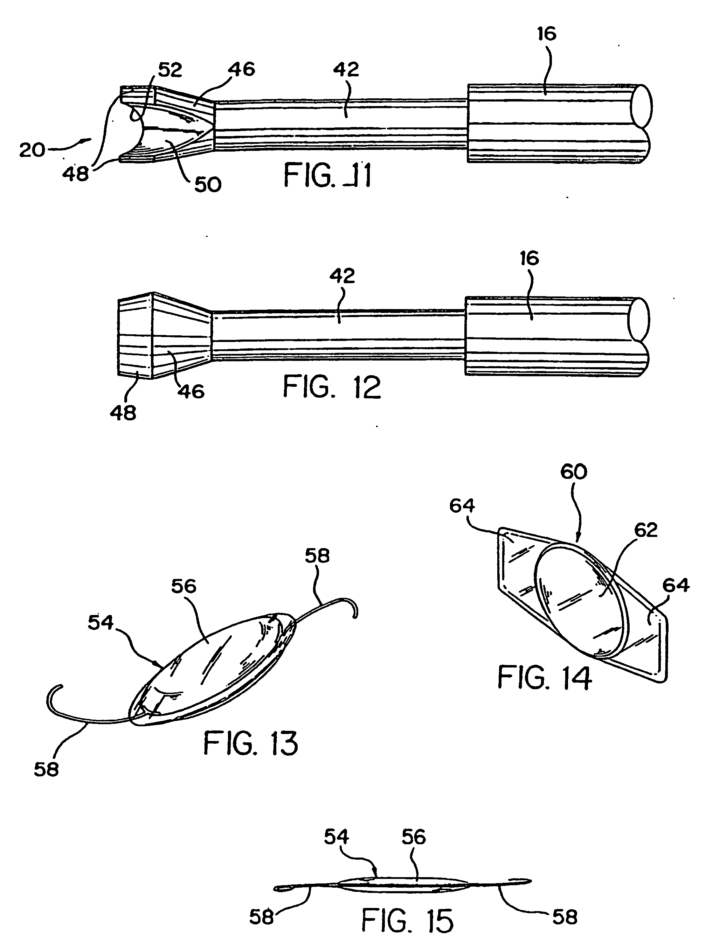

The details of the tip arrangement are shown in Figures 7 to 12. The plunger 16 is manufactured with an extension 42 supporting tip 20. The tip 20 structure provides means for inserting the deformable intraocular lens into the eye and manipulating the lens within the eye after the insertion step. For example, the tip 20 is faceted in the manner shown in the figures. Specifically, the left side of the tip 20 shown in Figure 8 is provided with a flat surface facet 44, conical surface 46, and cylindrical surface 48. The right side shown in Figure 11 is provided with a concave surface facet 50.

The end face of the tip 20 is designed to push the lens into position once inserted into the eye. For example, the end face is defined by a concave cylindrical surface 52 shown in Figure 8.

Suitable deformable intraocular lens for use in the present invention are shown in Figures 13 - 15. The deformable intraocular lens 54 shown in Figures 13 and 15 includes a lens body 56 with attachment means defined by a pair of haptics 58 each having one end anchored in the lens portion 56 and a free end for attachment to the eye tissue. The deformable intraocular lens 60 shown in Figure 14 includes a lens body 62 and attachment means defined by a pair of lateral lobes 64 of the lens portion 62.

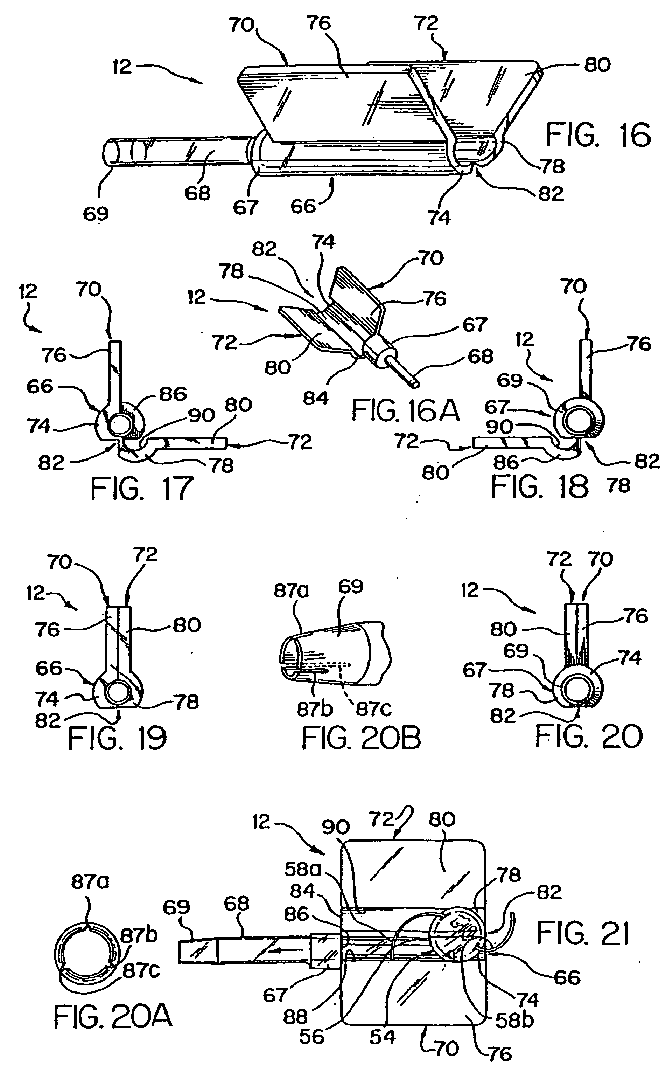

The details of the prior art lens holding microcartridge 12 are shown in Figures 16 - 20. The microcartridge 12 comprises a split tubular member 66 extending to a continuous tubular member 67 and an implantation nozzle 68. When the microcartridge is in a closed position, a continuous circular or oval passageway of the same diameter extends through the split tubular member 66 through the continuous tubular member 67 and through the implantation nozzle 68. The microcartridge is preferably made of injection molded plastic such as polypropylene The split tubular member 66 is defined by a fixed portion 70 and a moveable portion 72. The fixed portion 70 is fixed relative to the implantation nozzle 68, and is defined by a tubular portion 74 and extension 72. The moveable portion 72 is moveable relative to the fixed portion 70 for opening and closing the split tubular member 66. The moveable portion 72 is defined by a tubular portion 78 and extension 80. A hinge 82 is provided between the fixed portion 70 and moveable portion 72. The hinge 82 is defined by reducing the thickness of the walls of the tubular portion 74 and 75 at the hinge 82, as shown in Figures 17, 18 and 19. The hinge 82 runs the length of the split tubular member 66 to allow the extension 76 and 78 to be split apart, or brought together to open and close, respectively, the split tubular member 66.

The tubular portion 78 of the moveable portion 72 is provided with a sealing edge 84, which is exposed when the lens holding microcartridge 12 is opened, as shown in Figure 16A, and seals with a similar sealing edge 86 (See Figures 17 and 21) of the continuous tubular member 67 when the lens holding microcartridge is closed.

The end of the tip 20 is provided with three (3) equally spaced slots 87a, 87b and 87c of different length provided about the circumference thereof, as shown in Figures 20A and 20B. The slot 87a positioned at the top of the tip 20 is the shortest, slot 87c on the right side of the tip 20 is the longest, and slot 87b on the left side is of medium length. The slots 87a, 87b, 87c cause the lens 54 to rotate as it exits the tip 20.

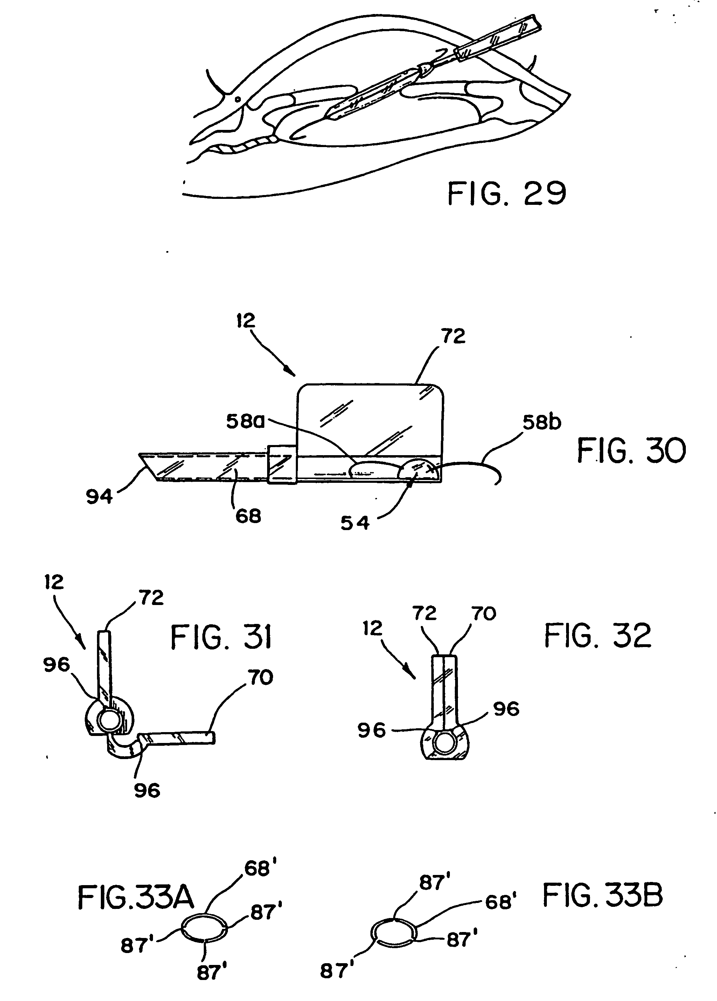

Other embodiments of the microcartridge 12 according to the present invention are shown in Figures 30-33.

The microcartridge shown in Figure 30 is provided with a beveled tip 94 to facilitate entry of the tip through the incision in the eye during implantation. The beveled tip 94 can be set at approximately forty-five (45) degrees relative to the passageway through the microcartridge 12.

The embodiment of the microcartridge shown in Figures 31 and 32 is provided with a set of grooves 96 provided inside the passageway therethrough. The grooves accommodate the edges of the lens being loaded into the microcartridge to facilitate bending of the lens. Specifically, the edges of the lens are placed in the grooves 96 to prevent relative slippage of the edges with the inner surface of the passageway through the microcartridge when the microcartridge is being folded into the closed position.

The embodiments of the microcartridge shown in Figures 33A and 33B each have a nozzle 68' having an oval cross-section with slots 87' differently position as shown, respectively, again to facilitate entry through an incision in the eye. Alternatively, the cross-section can be two half circles set apart and connected together rather than oval.

The various features of the microcartridges shown in Figures 16-21 and 30-33 can be used in various combinations to achieved an optimum design for a particular application. However, all of these features are typically considered improvements of the basic combination.

The components of the device 10, except for the microcartridge 12, are preferably fabricated from autoclavable material such as stainless steel or from a disposable rigid plastic such as medical grade ABS or the like.

### HINGELESS INTRAOCULAR LENS MICROCARTRIDGE

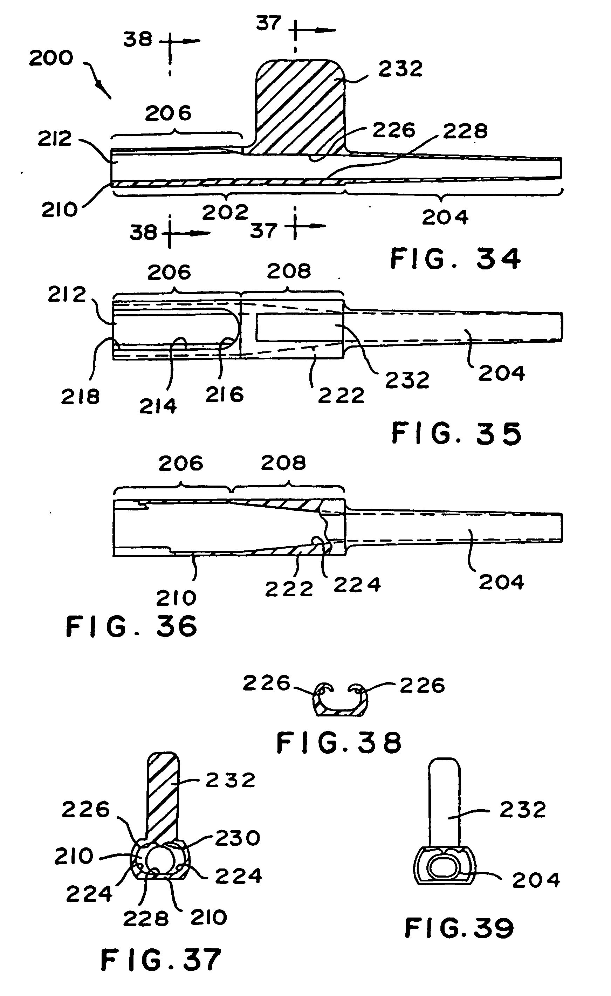

A hingeless type intraocular lens microcartridge according to the prior art are shown in Figures 34-43.

The lens microcartridge 200 comprises a lens holder portion 202 and a nozzle portion 204 connected to and extending from one end of the lens holder portion 202. The lens microcartridge is provided with a continuous passageway therethrough that extends from one end of the lens microcartridge to the other end thereof. The lens holder portion 202 includes a receiver portion 206 for receiving a deformable intraocular lens and a transition portion 208, as shown in Figs. 35 and 36.

The receiver portion 206 is defined by a tubular member 210 provided with an oval shaped barrel 212 having a longitudinal slot 214 therethrough. The oval shaped barrel 212 has a constant cross section or a gradually reducing cross section throughout the length of the receiver portion 206. Further, the longitudinal slot 214 has a rounded end 216 at one end, and an open end 218 at an opposite end thereof.

The transition portion 208 is defined by a tubular member 220 with an oval shaped barrel 222 having a cross section that tapers inwardly from the receiver portion 206 to the nozzle portion 204. Specifically, the sides 224 of oval shaped barrel 222 taper inwardly, as shown in Fig. 36, while the top surfaces 226 (i.e. defining grooves 226) and bottom surface 228 of the oval shaped barrel 222 are parallel (i.e. not tapered), as shown in Fig. 34. Further, the top 226 is provided with a downwardly extending protrusion 230 having curved sides that are shaped to turn the edges of the deformable intraocular lens downwardly, as shown in Fig. 37. The protrusion becomes less pronounced in a direction extending towards the nozzle portion 204, and disappears at the nozzle portion to a provide a continuous inner surface and transition from the lens holder 202 portion into the nozzle portion 204.

The grooves 226 in the receiver portion 206, as shown in Figure 38, are defined by curled upper portions of the receiver portion 206 on either side of the slot 214, and extend continuously to the grooves of the transition portion 208.

The lens microcartridge 200 is provided with an extension 232 for aligning the lens microcartridge 200 in the device for implantation of the intraocular lens. Specifically, the extension 232 is defined by plastic material that extends from the lens holder portion 202, and is configured to snag fit in the slot of the device for implantation of the intraocular lens. In the embodiment shown in Figs. 34-39, the extension 232 has a rectangular side profile (See Fig. 34) and has a constant thickness (See Fig. 35) along its length.

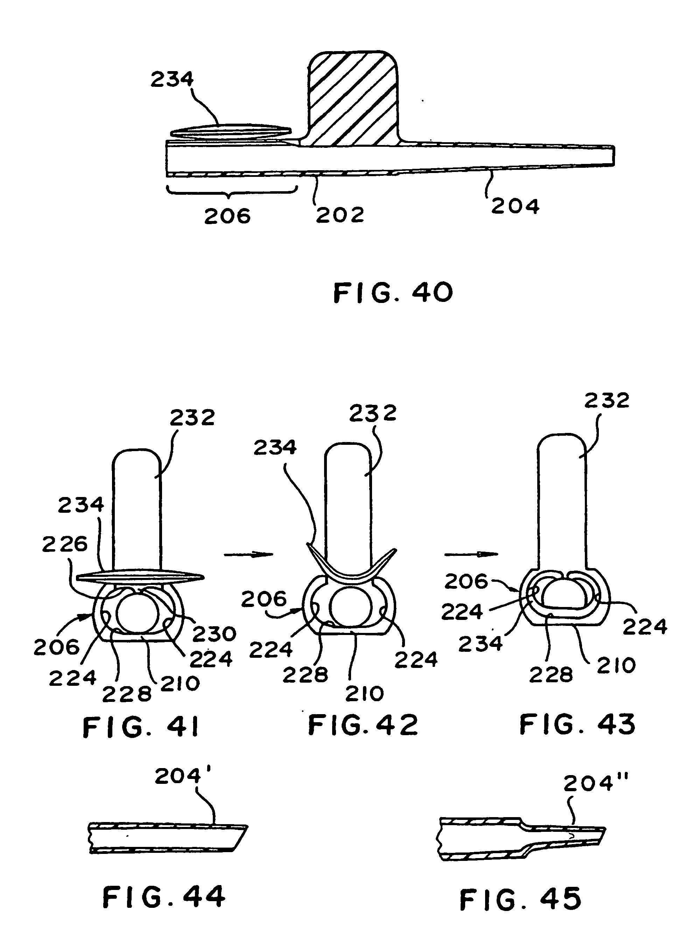

The manner in which the deformable intraocular lens is inserted into the lens microcartridge is illustrated in Figs. 40-43.

A deformable intraocular lens 234 is loaded on top of the receiver portion 206 of the lens holder portion 202. The deformable intraocular lens 234 is forced downwardly in the center thereof by implement or finger tip to reach the configuration shown in Fig. 42. The deformable intraocular lens is further pushed into the receiver portion 206 until it is fully inserted in the oval shaped configuration shown in Fig. 43 with its outer surface wrapping around and in contact with the sides 224, bottom 228, and top 226 of the receiver portion 206. The grooves defined by surfaces 226 hold the lens in position and guide it while the lens is being pushed through the microcartridge by the insertion instrument.

The edges of the deformable intraocular lens 234 contact with the downwardly extending protrusion 230 in the top 226, and ride along the protrusion 230 when being inserted through the lens microcartridge gently further folding the lens as it enters into the nozzle portion 204.

An alternative nozzle portion 204' is shown in Figure 44 having a beveled end. A further alternative nozzle portion 204" is shown in Figure 45 having a heat deformed tip with a beveled end. Specifically, the end of the nozzle is heated and stretch to reach the shape and configuration shown.

### METHODS OF IMPLANTATION

The surgical procedure begins by coating the lens with a surgically compatible lubricant, and loading the lens into the microcartridge. For example, as shown in Figure 21, a lens 54 having a lens body 56, a leading haptic 58a is loaded into a prior art microcartridge 12 while a trailing haptic 58b remains trailing outside the microcartridge in the manner shown. Specifically, the lens 54 is loaded downwardly into the opened microcartridge 12 until it sits on the inner surfaces of the tubular portions 74 and 78, for example, with a pair of tweezers. The outer circumferential surface of the lens 54 are held by edges 88 and 90 of the tubular portions 74 and 78, respectively. The rear edge of the lens 54 is placed approximately at the rear edge of the microcartridge 12. The lens 54 is further manipulated to situate the haptics 58a and 58b in the manner shown. Specifically, haptic 54a is positioned in a leading position and the other haptic 54b is positioned in a trailing position outside with respect to the direction of implantation, as indicated by the arrow.

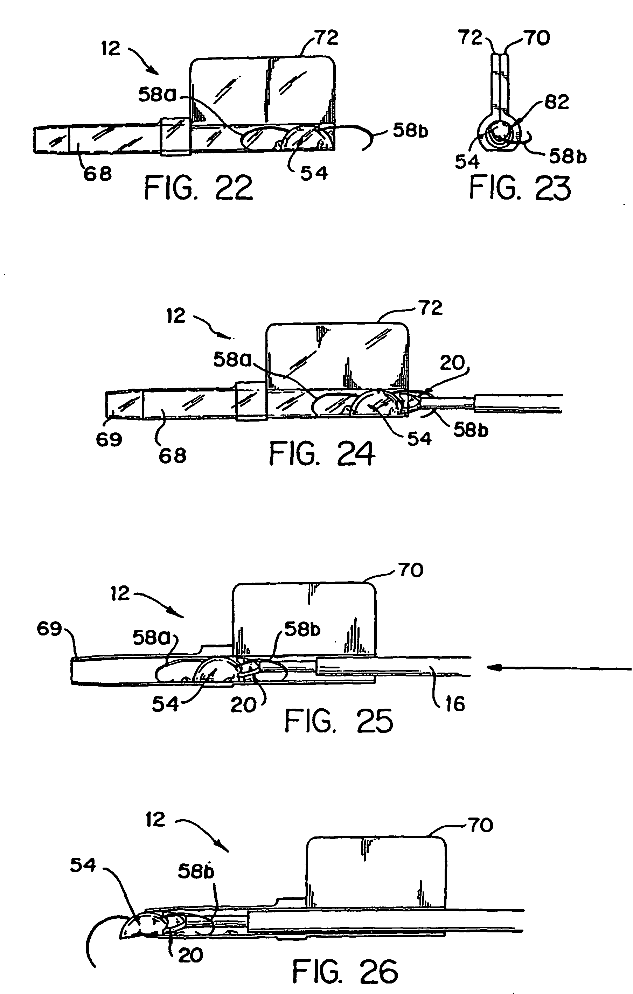

Subsequently, the split tubular member 66 of the microcartridge 12 is closed about the lens 54 by forcing the extensions 76 and 80 together with his or her finger tips. The inner surfaces of the tubular portions 74 and 78 bend and fold the lens 54 when the extensions 76 and 80 are forced together, as shown in Figures 22 and 23. Due to the resilient nature of the deformable intraocular lens 54, the lens 54 conforms to the curved inner surface of the tubular portions 74 and 78 without damage thereto, as shown in Figure 23.

The microcartridge 12 containing the loaded lens 54 is inserted between the edges 17a, 17a of the opening 17 into the receiver 15 of the holder 13. As the microcartridge 12 is moved forward, the extensions 76 and 80 move past the tapered edges 17b and come to a stop position between the clamping edges 17c when front portions of the extensions 76 and 80 contact with the stop edge 17d. The clamping edges 17c prevent rotation of the microcartridge inside the holder 13.

The user pushes the threaded end cap 18 forward while securing the holder body 14 from movement, forcing the plunger 16 forward within the holder. As the plunger 16 is moved forward, the tip 20 enters into the rear of the microcartridge 12 and misses the trailing haptic 58B until the tip makes contact with the loaded lens 54, as shown in Figure 24. As the plunger 16 is moved forward in this manner, the lens 54 previously lubricated, is forced into the implantation nozzle 68 of the microcartridge 12, as shown in Figure 25.

Once the lens 54 enters the implantation nozzle 68, the threads of the end cap 18 contact with the threads of the sleeve 24 stopping further movement of the plunger 14 forward in this manner. The end cap 18 is slightly rotated to engage the threads of the end cap 18 with the threads of the sleeve 24. At this point, the surgical device is ready for the implantation step. The nozzle is inserted through the incision in the eye, and the end cap 18 is rotated to continue the forward movement of the plunger 16 by continued rotation of the end cap 18 relative to the holder body 14 to expel the lens from the nozzle into the interior of the eye, as shown in Figure 26. This manner of screw advancement for moving the plunger 16 forward provides for precise control and accuracy concerning forcing the lens 54 through the remaining portion of the tip 68 into the eye during the implantation procedure. The deformed lens after exiting the nozzle 16 returns to its original configuration, full size and fixed focal length.

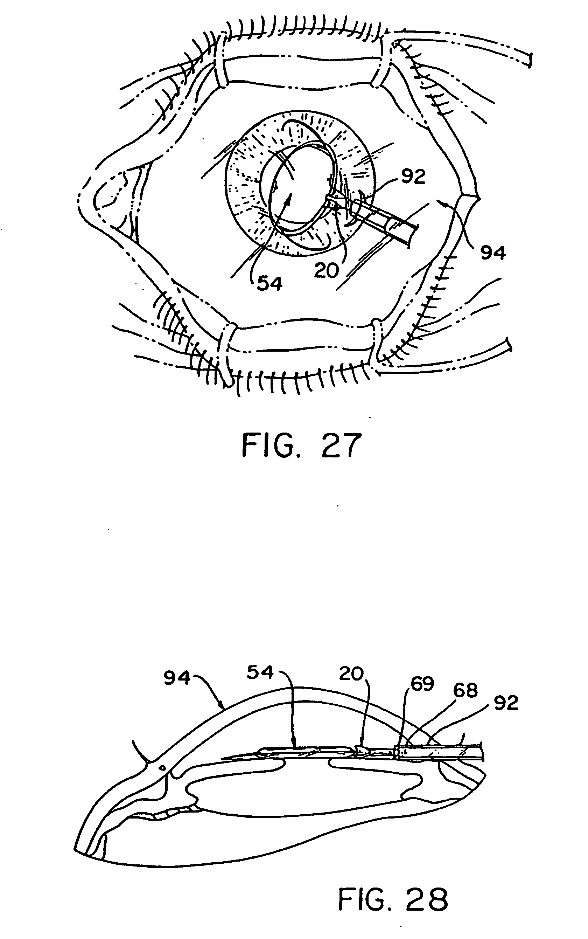

After the lens is inserted into the eye, the end cap 18 is further rotated to fully expose the tip 20 of the plunger 16, as shown in Figures 28 and 29, to allow the lens to be pushed forward, side manipulated to rotate the lens, and pushed down to properly position the lens within the eye without the aid of other surgical instruments.

The configuration of the tip 20 is important during the implantation process. The faceted tip 20 provides a clearance between the tip 20 and the inner surface of the passageway through the microcartridge 12 to accommodate the trailing haptic 58b during movement of the lens within the microcartridge 12, as shown in Figures 25 and 26. Specifically, there exists a sufficient clearance between the flat surface facet 44 and the inner wall of the passageway through the microcartridge 12. During the implantation process, the trailing haptic floats around in the space between the extension 42 of the tip 20 and the inner wall of the passageway, as shown in Figure 25. This prevents any chance of damage to the trailing haptic, for example, by being caught between the tip 20 and the lens 54 during the implantation process. The leading haptic moves through the passageway unimpeded during the implantation process preventing any damage thereto.

### FLAT LOADING LENS CARTRIDGE

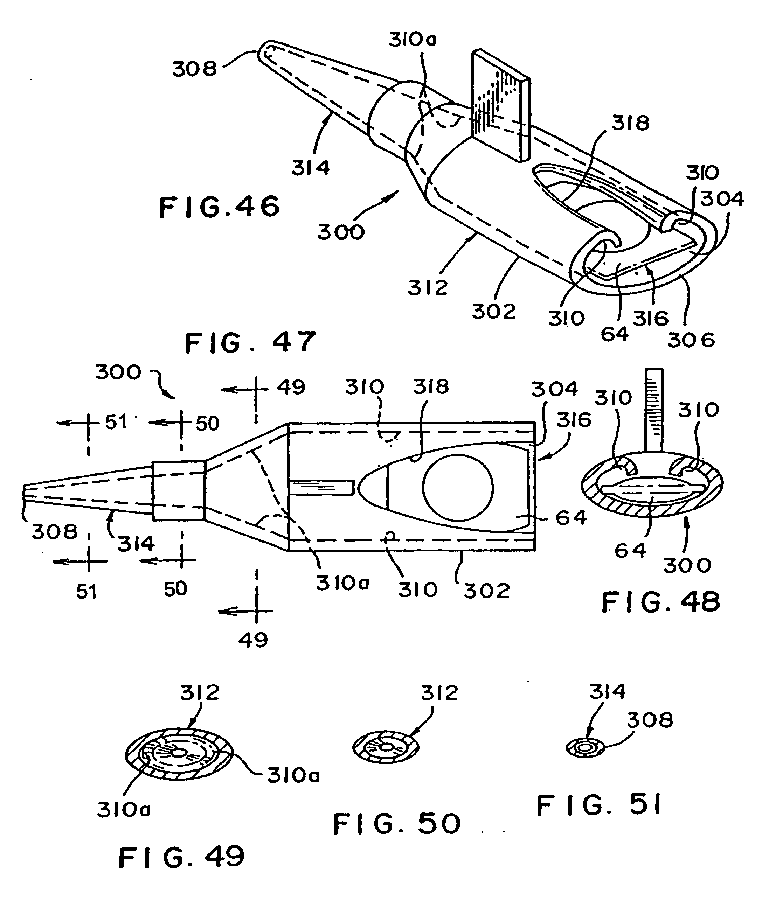

A preferred embodiment of a cartridge 300 according to the invention is shown in Figures 46 to 51.

The cartridge 300 is a contoured duct apparatus for curling and inserting a flexible intraocular lens into an eye with low radial pressure. The cartridge 300 is defined by a generally tubular body 302 provided with a longitudinally disposed lens-curling duct 304 having a lens inlet end 306 and lens outlet end 308 for insertion into an eye. The lens inlet end 306 and lens outlet end 308 are connected by a pair of mutually opposed converging longitudinal lens-engaging grooves 310 for guiding opposing edge portions of the lens towards one another with low radial pressure to a curled position as the lens is advanced through the duct 304 from the inlet end 306 to the outlet end 308.

The cartridge 300 can be configured to load in the injecting device shown in Figure 1. The plunger 16 of the injecting device provides means for advancing the lens through the duct 304 of the cartridge 300 from the inlet end 306 to the outlet end 308.

The lens inlet end 306 is an elongated transverse aperture having a major axis, and wherein proximal ends of the lens engaging grooves 310 are disposed at opposing ends of the major axis to slidingly engage diametrically opposed peripheral edge portions of the lens. The opposing longitudinal grooves 310 include portions 310a that converge from the lens inlet end 306 to the lens outlet end 308.

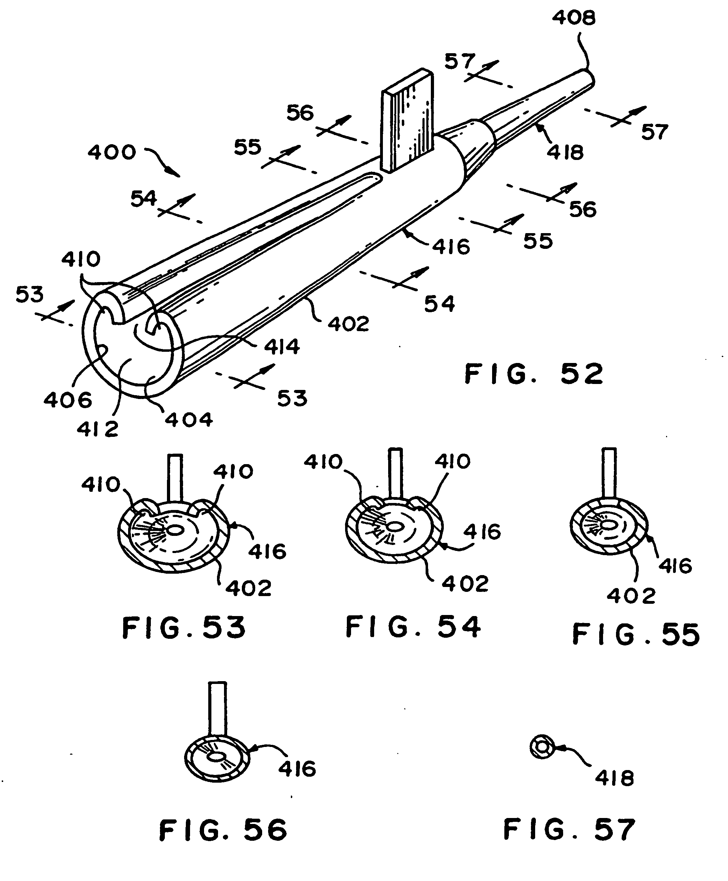

The lens outlet end 306 can have an oval cross-sectional shape, as shown in Figure 51, or alternatively can have a generally circular transverse aperture. Further, the inlet end 306 and the outlet end 308 are coaxially aligned along a longitudinal axis of the duct. The means for advancing the lens is a coaxially aligned plunger 16 of the injecting device shown in Figure 1 longitudinally disposed within the tubular body during lens injection, and provided with a lens engaging tip 20. The lens engaging tip 20 is provided with a lens haptic receiving relief.

The cartridge 300 is defined by a lens holding portion 312 and a nozzle portion 314. The nozzle portion 314 can act the same as or equivalent to a curled lens insertion cannula that is disposed in coaxial lens conducting communication with the lens outlet end 308. The tubular body 302 is provided with a lens receiving chamber 316 disposed adjacent to the lens inlet end 306. In addition, the lens receiving chamber 316 is provided with a slot 318 or peripheral lens access opening.

### FUNNEL TYPE CARTRIDGE

A further embodiment of a cartridge 400 according to the invention shown in Figures 52 to 57.

The cartridge 400 is defined by a generally tubular body 402 provided with a longitudinally disposed lens-curling duct 404 having a longitudinal axis, an inlet end 406 with a transverse major axis, and generally circular outlet end 408 longitudinally aligned with said inlet end 406. The duct 404 includes a pair of mutually opposed lens engaging grooves 410 with proximal ends disposed at opposite ends of said major axis and diametrically opposed peripheral edges of the lens. The pair of mutually opposed lens engaging and guiding grooves 410 converge toward the outlet end 408 and terminate above a plane determined by the perpendicular intersecting of the inlet major axis with the duct longitudinal axis. A lens chamber 412 is disposed adjacent to the inlet end 406 provided with a peripheral slot 414 or peripheral opening for access to the interior of the chamber 412.

The cartridge 400 can be loaded in the injecting device shown in Figure 1. The plunger 16 of the injecting device is longitudinally disposed within the tubular body 402 during lens injection, and is aligned coaxially with the longitudinal axis of the lens-curling duct 404.

The cartridge 400 is defined by a lens holding portion 416 and a nozzle portion 418. The nozzle portion 418 can function equivalent to a cannula coaxially aligned with the duct 404.

## Claims

1. A contoured duct apparatus for curling and inserting a flexible intraocular lens (54, 60) into an eye with low radial pressure, said apparatus comprising:
a holder (13) having a receiver (15) at one end thereof;
a moveable plunger (16) disposed within the holder (13); and
a lens holder (300, 400) which is adapted to be removably inserted in said receiver of said holder (13), the lens holder (300, 400) having a generally tubular body (302, 402) provided with a longitudinally disposed contoured lens curling duct (304,404) having a lens inlet end (306, 406) and an outlet end (308, 408), **characterised in that** said lens inlet en and outlet end are connected by a pair of mutually opposed converging longitudinal lens engaging grooves (310, 410) for guiding opposing edge portions of a lens toward one another with low radial pressure to a curled position as the lens is advanced through said duct (304, 404) from said lens inlet end (306, 406) to said outlet end (308, 408) by said plunger, wherein said lens inlet end (306, 406) is an elongated transverse aperture having a major axis wherein proximal ends of said lens engaging grooves are disposed at opposing ends of said major axis to slidingly engage diametrically opposed peripheral edge portions of said lens, said opposing longitudinal grooves converging from said lens inlet end (306, 406) to said outlet end (308, 408) toward one another and to a position above said major axis.

2. Apparatus according to claim 1, wherein said outlet end (308, 408) is a generally circular transverse aperture.

3. Apparatus according to claim 1 or 2, wherein said lens inlet end and said outlet end are coaxially aligned along a longitudinal axis of said lens holder.

4. Apparatus according to any of claims 1 to 3, wherein said plunger is coaxially aligned and longitudinally disposed within said tubular body and provided with a lens engaging tip (20).

5. Apparatus according to claim 4, wherein said lens engaging tip is provided with a lens haptic receiving relief.

6. Apparatus according to any of claims 1 to 5, further comprising a curled lens insertion cannula (314, 418) disposed in coaxial lens conducting communication with said outlet end.

7. Apparatus according to any of claims 1 to 6, wherein said tubular body is provided with a lens receiving chamber (316, 412) disposed adjacent to said lens inlet end.

8. Apparatus according to claim 7, wherein said lens-receiving chamber is provided with a peripheral lens access opening (318, 414).

## Patentansprüche

1. Ein konturierter Führungsapparat zum Einrollen und Einführen einer flexiblen intraokularen Linse (54, 60) in ein Auge mit geringem radialem Druck, die Vorrichtung umfassend:
einen Halter (13), der an einem Ende eine Aufnahme (15) aufweist;
einen bewegbaren Kolben (16), der in dem Halter 13 angeordnet ist; und
einen Linsenhalter (300, 400), der ausgebildet ist, um entfembar in die Aufnahme des Halters (13) eingeführt zu werden, wobei der Linsenhalter (300, 400) einen allgemein rohrförmigen Körper (302, 402) aufweist, der mit einer longitudinal angeordneten, konturierten Linseneinrollkanal (304, 404) versehen ist, der ein Linseneinlassende (306, 406) und ein Auslassende (308, 408) aufweist, **dadurch gekennzeichnet, dass** das Linseneinlassende und das Auslassende durch ein Paar von zueinander gegenüberliegenden, konvergierenden longitudinalen auf die Linse einwirkenden Nuten (310, 410) verbunden sind zum Führen gegenüberliegender Kantenabschnitte einer Linse aufeinander zu mit geringem radialen Druck zu einer eingerollten Position, wenn die Linse durch den Kanal (304, 404) von dem Linseneinlassende (306, 406) zu dem Auslassende (308, 408) durch den Kolben gefördert wird, worin das Linseneinlassende (306, 406) eine längliche Queröffnung ist, die eine Hauptachse aufweist, worin proximale Enden der mit der Linse zusammenwirkenden Nuten an gegenüberliegenden Enden der Hauptachse angeordnet sind, um verschieblich diametral gegenüberliegende Umfangskantenabschnitte der Linse zu erfassen, wobei die gegenüberliegenden, longitudinalen Nuten von dem Einlassende (306, 406) zu dem Auslassende (308, 408) aufeinander zu und zu einer Position über der Hauptachse konvergieren.

2. Apparat nach Anspruch 1, worin das Auslassende (308, 408) eine grundsätzlich kreisförmige Queröffnung ist.

3. Apparat nach Anspruch 1 oder 2, worin das Linseneinlassende und das Aus assende koaxial entlang einer longitudinalen Achse des Linsenhalters ausgerichtet sind.

4. Apparat nach einem der Ansprüche 1 bis 3, worin der Kolben koaxial ausgerichtet und longitudinal innerhalb des rohrförmigen Körpers angeordnet ist und mit einer mit der Linse zusammenwirkenden Spitze (2) versehen ist.

5. Apparat nach Anspruch 4, worin die mit der Linse zusammenwirkende Spitze mit einem linsenhaptischen Aufnahmerelief versehen ist.

6. Apparat nach einem der Ansprüche 1 bis 5, weiter umfassend eine Einführungskanüle (314, 418) für eine eingerollte Linse, die in koaxialer Linsenführungsverbindung mit dem Auslassende angeordnet ist.

7. Apparat nach einem der Ansprüche 1 bis 6, worin der rohrförmige Körper mit einer Linsenaufnahmekammer (316, 412) versehen ist, die benachbart zu dem Linseneinlassende angeordnet ist.

8. Vorrichtung nach Anspruch 7, worin die Linsenaufnahmekammer mit einer peripheren Linsenzugangsöffnung (318, 414) versehen ist.

## Revendications

1. Appareil à conduit profilé pour incurver et insérer un cristallin artificiel flexible (54, 60) dans un oeil avec une faible pression radiale, ledit appareil comprenant :
une monture (13) ayant un organe récepteur (15) à une extrémité de celle-ci;
un plongeur mobile (16) disposé à l'intérieur de la monture (13) ; et
un support de cristallin (300, 400) qui est adapté pour être inséré de manière amovible dans ledit organe récepteur de ladite monture (13), le support de cristallin (300, 400) ayant un corps globalement tubulaire (302, 402) pourvu d'un conduit (304, 404), d'incurvation de cristallin profilé et agencé longitudinalement, ayant une extrémité d'entrée (306, 406) et une extrémité de sortie (308, 408) de cristallin, **caractérisé en ce que** lesdites extrémité d'entrée et extrémité de sortie de cristallin sont reliées par une paire de rainures (310, 410) de mise en prise de cristallin longitudinales, convergentes et mutuellement opposées, destinées à guider des parties de bord opposées d'un cristallin l'une vers l'autre avec une faible pression radiale jusqu'à une position incurvée au fur et à mesure que le cristallin est avancé par ledit plongeur à travers ledit conduit (304, 404) depuis ladite extrémité d'entrée (306, 406) jusqu'à ladite extrémité de sortie (308, 408) de cristallin, dans lequel ladite extrémité d'entrée (306, 406) de cristallin est une ouverture transversale allongée ayant un grand axe, dans lequel des extrémités proximales desdites rainures de mise en prise de cristallin sont disposées à des extrémités opposées dudit grand axe afin de mettre en prise de manière coulissante des parties de bord périphériques diamétralement opposées dudit cristallin, lesdites rainures longitudinales opposées convergeant l'une vers l'autre depuis ladite extrémité d'entrée (306, 406) jusqu'à ladite extrémité de sortie (308, 408) de cristallin et jusqu'à une position au-dessus dudit grand axe.

2. Appareil selon la revendication 1, dans lequel ladite extrémité de sortie (308, 408) est une ouverture transversale globalement circulaire.

3. Appareil selon la revendication 1 ou 2, dans lequel ladite extrémité d'entrée et ladite extrémité de sortie de cristallin sont alignées coaxialement le long d'un axe longitudinal dudit support de cristallin.

4. Appareil selon une quelconque des revendications 1 à 3, dans lequel ledit plongeur est aligné coaxialement et disposé longitudinalement à l'intérieur dudit corps tubulaire, et est pourvu d'un embout de positionnement de cristallin (20).

5. Appareil selon la revendication 4, dans lequel ledit embout de positionnement de cristallin est pourvu d'un relief de réception haptique de cristallin.

6. Appareil selon une quelconque des revendications 1 à 5, comprenant en outre une canule d'insertion de cristallin incurvée (314, 418), agencée en communication de guidage coaxial de cristallin avec ladite extrémité de sortie.

7. Appareil selon une quelconque des revendications 1 à 6, dans lequel ledit corps tubulaire est pourvu d'une chambre de réception de cristallin (316, 412), disposée de façon adjacente à ladite extrémité d'entrée de cristallin.

8. Appareil selon la revendication 7, dans lequel ladite chambre de réception de cristallin est pourvue d'une ouverture périphérique d'accès au cristallin (318, 414).
